# EUROPÄISCHE PATENTSCHRIFT

(11) **EP 1 107 776 B1**
(45) Veröffentlichungstag und Bekanntmachung des Hinweises auf die Patenterteilung: **11.02.2004**
(21) Anmeldenummer: 99940194.6
(22) Anmeldetag: 10.08.1999
(51) Int. Cl.: A61K 35/78, A61K 7/48, A61K 7/13

(54) **TROCKENEXTRAKT AUS DER HENNAPFLANZE, VERFAHREN ZU DESSEN HERSTELLUNG UND DESSEN VERWENDUNG**
DRY EXTRACT OF HENNA, METHOD FOR PRODUCING SAID EXTRACT AND USE OF THE SAME
EXTRAIT SEC DE HENNE, PROCEDE PERMETTANT DE L'OBTENIR ET SON UTILISATION

(30) Priorität: 26.08.1998 DE 19838809
(43) Veröffentlichungstag der Anmeldung: 20.06.2001
(73) Patentinhaber: Kloss, Gerhard, 31008 Elze (DE)
(72) Erfinder: WEILAND-GROTERJAHN, Heinz-Jürgen, D-31789 Hameln (DE); PAPE, Andreas, D-32694 Dörentrup (DE); WÄHLING, Axel, D-39120 Magdeburg (DE); LANGE, Elfriede, D-39126 Magdeburg (DE)
(74) Vertreter: Grünecker, Kinkeldey, Stockmair & Schwanhäusser Anwaltssozietät
(86) Internationale Anmeldenummer: PCT/EP1999/005838
(87) Internationale Veröffentlichungsnummer: WO 2000/012108

(56) Entgegenhaltungen:
- DE-A- 3 744 352
- FR-A- 2 473 310
- PATENT ABSTRACTS OF JAPAN vol. 1996, no. 07, 31. Juli 1996 (1996-07-31) & JP 08 081342 A (NIPPON FINE CHEM CO LTD), 26. März 1996 (1996-03-26)

## Beschreibung

Die Erfindung betrifft einen Trockenextrakt aus der Hennapflanze oder Teilen davon, ein Verfahren zur dessen Herstellung und dessen Verwendung sowie eine Emulsion, diesen enthaltend.

Die Hennapflanze (*Lawsonia inermis* Linn, *Lawsonia alba, Lawsonia spinosa*) ist ein in den Tropen und Subtropen weit verbreiteter Busch, dessen Blätter seit Tausenden von Jahren als Medizin, als Kosmetikum zum Anfärben von Haaren, zum Bemalen von Händen und Füßen und auch zum Anfärben von Textilien verwendet werden. Die färbenden Eigenschaften der Hennapflanze (im nachstehenden "Henna") rühren von Lawson (2-Hydroxy-1,4-naphthochinon (I)) her, das zuerst von Tommasi isoliert und identifiziert wurde (Tommasi, G., Gazz. *Chim. et al*., **50** (1920), 263).

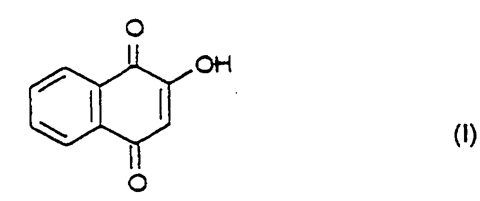

Der höchste Gehalt an Lawson wird in den Hennablättern gefunden. Dieser Gehalt beträgt etwa 1% Lawson. Außerdem enthält Henna etwa 10% Gerbstoffe und wenig freie Gallussäure.

Herkömmlicherweise werden zermahlene Hennablätter zum Färben von Haaren verwendet. Diese werden mit ausreichend heißem Wasser zur Bildung einer glatten Paste gemischt und in Abhängigkeit vom gewünschten Effekt und der Intensität der Färbung 15 bis 60 Minuten auf das Haar aufgebracht. Beispielsweise können zur Herstellung einer solchen Paste ein Eßlöffel zerriebene Hennablätter mit 250 ml heißem Wasser gemischt werden. Diese Paste wird warm auf die Haare aufgebracht und dort 10 bis 20 Minuten belassen. Sodann wird sie mit heißem Wasser ausgewaschen (Botanicals in Cosmetics, Band 98, Juni 1983, Seite 91). Des weiteren ist es bekannt, flüssige Extrakte von zerkleinerten Hennablättern zum Färben von Wolle zu verwenden. Dazu werden zerkleinerte Hennablätter mit 0,1 molarer Natriumhydroxidlösung oder destilliertem Wasser 48 Stunden lang bei Raumtemperatur gerührt. Das entstehende Gemisch wird unter Vakuum filtriert. Der Lawsongehalt des alkalischen flüssigen Extrakts wurde mittels HPLC (high pressure liquid chromatography) bestimmt, wobei ein durchschnittlicher Lawsongehalt von 0,75% festgestellt wurde. Außerdem wurde Wolle mit dem neutralen flüssigen Extrakt bei verschiedenen pH-Werten angefärbt, wobei gefunden wurde, daß der Aufnahmegrad von Hennaextrakt durch die Wolle mit steigendem pH-Wert abnimmt (Dyes and Pigments 22 (1993), 15 bis 25).

Außerdem ist ein flüssiger Extrakt von zerkleinerten Hennablättern bekannt, der durch Mazerieren mit einer 5% Natriumhydrogencarbonatlösung für 24 Stunden, Perkolieren mit 700 ml Alkohol und Konzentrieren des flüssigen Extrakts auf 100 ml erhalten wird. Dieser kann in einer Hennasalbe auf der Basis von Polyethylenglykol zur Behandlung von Verbrennungen verwendet werden (Indien Y. Pharm. Sci., 58 (2), (1996), 55 bis 58).

Außerdem ist ein Verfahren zur Herstellung von Lawson bekannt, bei dem die zerkleinerten Hennablätter mit gesättigter Natriumcarbonatlösung extrahiert werden, sodann mit HCl angesäuert wird und mit einer Vorrichtung zur Flüssig-Flüssig-Extraktion mit Chloroform extrahiert wird. Der Chloroformextrakt wird konzentriert und man erhält Lawson, das durch Umkristallisieren mit Methanol-Benzol (1:1) gereinigt wird.

FR-2 473 310 verwendet organische Lösungsmittel wie Dichlorethan zur Herstellung von Extrakten aus der Hennapflanze.

Um toxikologische Risiken zu vermeiden, ist in jüngster Zeit ein gestiegenes Bestreben festzustellen, Naturfarbstoffe anstelle von synthetischen Farbstoffen zu verwenden. Wegen ihres schwachen Aufziehvermögens weisen viele der bekannten natürlichen Farbstoffe jedoch nur geringe Färbeeigenschaften auf. Außerdem ist beispielsweise die Konzentration der farbgebenden Komponente Lawson in den bekannten Extrakten von Henna und den zermahlenen Hennablättern sehr gering.

Wegen des gestiegenen ökologischen Bewußtseins in weiten Teilen der Bevölkerung, besteht außerdem ein Bedarf, auf ökologisch nicht verträgliche Substanzen, wie organische Lösungsmittel, bei der Herstellung von Produkten zu verzichten.

Daher ist es Aufgabe der vorliegenden Erfindung, einen ökologisch unbedenklichen Trockenextrakt aus der Hennapflanze bereitzustellen, der verbesserte Färbeeigenschaften aufweist. Des weiteren ist es Aufgabe der vorliegenden Erfindung, ein Verfahren zur Herstellung des Trockenextrakts bereitzustellen, bei dem keine ökologisch bedenklichen Substanzen verwendet werden.

Eine weitere Aufgabe der vorliegenden Erfindung ist es, ein Haarfärbemittel mit verbesserten Färbeeigenschaften auf der Basis des natürlichen Färbemittels Henna bereitzustellen, das in seiner Handhabung verbraucherfreundlicher ist.

Diese Aufgabe wird durch einen Trockenextrakt aus der Hennapflanze oder Teilen davon, enthaltend 10 bis 60 Gew.-% Lawson, gelöst welcher nach einem Verfahren hergestellt wird, bei dem keine ökologisch bedenklichen Substanzen verwendet werden. Vorzugsweise enthält der Trockenextrakt 20 bis 45 Gew.-%, insbesondere 35 bis 42 Gew.-% Lawson. Außerdem kann der Extrakt als weitere Bestandteile Gerbsäuren, Zucker, Mineralstoffe und Wasser enthalten.

Als Pflanzenmaterial zur Herstellung des Trockenextrakts aus der Hennapflanze können alle geeigneten Teile der Hennapflanze verwendet werden. Vorzugsweise werden das Perikarp, die Samen oder die Blätter oder deren Gemische, insbesondere die Blätter verwendet. Außerdem kann das Pflanzenmaterial zerkleinert, vorzugsweise zerschnitten, vermahlen oder pulverisiert verwendet werden.

Ein weiterer Gegenstand der vorliegenden Erfindung ist das Verfahren zur Herstellung des erfindungsgemäßen Trockenextrakts. Das erfindungsgemäße Verfahren umfaßt die Schritte:
(1) Mischen des Pflanzenmaterials der Hennapflanze oder Teilen davon mit einer sauren wäßrigen Lösung, wodurch eine Maische erhalten wird,
(2) Trennen der in Schritt (1) erhaltenen Maische in einen Trester und eine wäßrige Lösung,
(3) Extrahieren des in Schritt (2) erhaltenen Tresters mit einer alkalischen wäßrigen Lösung,
(4) Abtrennen des in Schritt (3) erhaltenen Extrakts von dem Trester,
(5) Konzentrieren des in Schritt (4) erhaltenen Extrakts,
(6) Abkühlen und Ansäuern des in Schritt (5) erhaltenen Extrakts, wodurch ein Lawsonschlamm erhalten wird, und
(7) Abtrennen und Trocknen des in Schritt (6) erhaltenen Lawsonschlamms.

Vorzugsweise kann das in Schritt (1) eingesetzte Pflanzenmaterial vor seiner Verarbeitung gesiebt werden, um Staubbestandteile mit einem Durchmesser von < 0,25 mm abzutrennen, da kommerziell erhältliches Pflanzenmaterial häufig mit Staubbestandteilen verunreinigt ist. Wenn das Pflanzenmaterial ungesiebt eingesetzt wird, kann zur Abtrennung der Staubbestandteile der in Schritt (4) erhaltene Extrakt filtriert werden, vorzugsweise mit einem Vibrationssieb, oder die Staubbestandteile können über Sedimentieren abgetrennt werden.

Das Pflanzenmaterial wird in Schritt (1) mit einer sauren wäßrigen Lösung gemischt. Vorzugsweise ist die saure wäßrige Lösung eine wäßrige HCl-Lösung mit einem pH-Wert von 1 bis 6, vorzugsweise 2,5. Das Verhältnis von saurer wäßriger Lösung zu Pflanzenmaterial ist vorzugsweise im Bereich von 1:1 bis 10:1, bevorzugt 2:1 bis 5:1 und insbesondere 3:1, bezogen auf die Menge an eingesetztem Pflanzenmaterial. Das Mischen wird über einen Zeitraum von 10 Minuten bis 3 Stunden, vorzugsweise 20 Minuten bis 1 Stunde und insbesondere 30 Minuten durchgeführt. Die Temperatur in Schritt (1) ist vorzugsweise Raumtemperatur. Außerdem kann zur Beschleunigung des Stoffübergangs während des Durchführens von Schritt (1) gerührt werden.

In Schritt (1) wird ein Großteil der wasserlöslichen Komponenten, wie Zucker, Eiweiß und Salze, entfernt. Das Ansäuern der wäßrigen Lösung dient dazu, den Verlust an Lawson möglichst gering zu halten, da Lawson im sauren Milieu nicht gut löslich ist. Man erhält eine Maische, die ein Gemisch aus einem Trester und einer wäßrigen Lösung ist.

In Schritt (2) wird der in Schritt (1) erhaltene Trester von der wäßrigen Lösung abgetrennt. Das Abtrennen kann vorzugsweise mit einem Dekanter oder einer Bandpresse, insbesondere mit einer Bandpresse, durchgeführt werden. Dabei fällt ein Extrakt mit einem pH-Wert von etwa 4 und einem gravimetrischen Trockensubstanzgehalt von etwa 8% an. Man erhält einen Trester.

In Schritt (3) wird der in Schritt (2) erhaltene Trester mit einer alkalischen wäßrigen Lösung extrahiert. Vorzugsweise ist die alkalische wäßrige Lösung eine 0,05 bis 0,5%ige, insbesondere eine 0,15%ige wäßrige NaOH-Lösung. Das Verhältnis von alkalischen wäßrigen Lösungen zu Trester ist vorzugsweise im Bereich von 1:1 bis 20:1, bevorzugt 5:1 bis 15:1, und insbesondere 10:1, bezogen auf die Menge an eingesetztem Pflanzenmaterial.

Die Extraktion kann 1 bis 5 Stunden, vorzugsweise 3 Stunden durchgeführt werden, wobei die Temperatur des Extraktionsmittels zwischen etwa 20 bis etwa 70°C, insbesondere bei 50°C liegt. Vorzugsweise kann die Extraktion auch unter Rühren oder Umpumpen des Extraktionsmittel erfolgen, da so der Stoffaustausch intensiviert und die Extraktion beschleunigt wird. Man erhält einen Extrakt.

In Schritt (4) wird der in Schritt (3) erhaltene Extrakt von dem Trester abgetrennt. Das Abtrennen wird zweckmäßigerweise mittels Abpressens, beispielsweise mit einer Bandpresse, oder durch Dekantieren durchgeführt. Dadurch wird ein Extrakt und ein Trester erhalten.

Wahlweise kann der in Schritt (4) abgetrennte Trester in einem Schritt (4) (a) mit Wasser nachextrahiert werden. Das Verhältnis von Wasser zu Trester liegt vorzugsweise im Bereich von 3:1 bis 10:1, insbesondere bei 5:1, bezogen auf die Menge an eingesetztem Pflanzenmaterial. Die Extraktion erfolgt bei der gleichen Temperatur wie in Schritt (4). Die Extraktion wird vorzugsweise für einen Zeitraum von 1 bis 5 Stunden, insbesondere 2 Stunden, durchgeführt. Der in Schritt (4) (a) erhaltene Extrakt kann gegebenenfalls mit dem in Schritt (4) erhaltenen Extrakt vereinigt und gemeinsam weiter verarbeitet werden.

Sodann wird der erhaltene Extrakt in einem Schritt (5) konzentriert. Das Konzentrieren kann vorzugsweise durch thermisches Konzentrieren oder mittels Umkehrosmose durchgeführt werden. Falls das Konzentrieren durch thermisches Konzentrieren durchgeführt wird, liegt die Temperatur vorzugsweise bei etwa 20 bis etwa 70°C, insbesondere bei etwa 50°C. Der Konzentrierungsgrad liegt vorzugsweise bei etwa 1:10 bis 1:20, insbesondere bei 1:15. Man erhält einen konzentrierten Extrakt.

Der in Schritt (5) erhaltene konzentrierte Extrakt wird in einem Schritt (6) abgekühlt und angesäuert. Der Extrakt wird vorzugsweise auf eine Temperatur von etwa 10 bis etwa 16°C, insbesondere auf etwa 15°C, gekühlt. Das Kühlen kann vorzugsweise mittels eines Wärmetauschers, beispielsweise eines Plattenwärmetauschers, durchgeführt werden. Durch das Kühlen wird die Löslichkeit von Lawson in dem Extrakt erniedrigt.

Der pH-Wert des Extrakts wird vorzugsweise mit HCl, vorzugsweise auf einen pH-Wert von etwa 2 bis etwa 6, insbesondere 2,5, gesenkt, da Lawson im sauren Milieu schlecht löslich ist. Schritt (6) kann während eines Zeitraums von 30 Minuten bis 5 Stunden, bevorzugter von 1 bis 3 Stunden und insbesondere 1,5 Stunden , durchgeführt werden, wobei der Extrakt vorzugsweise während dessen gerührt wird. Man erhält einen Lawsonschlamm.

In Schritt (7) wird der in Schritt (6) erhaltene Lawsonschlamm abgetrennt und getrocknet. Das Abtrennen wird vorzugsweise mittels eines Separators durchgeführt und das Trocknen mittels eines Vakuumtrockners. Dadurch wird ein Trockenextrakt mit einem Gehalt von 10 bis 60 Gew.-% Lawson erhalten.

Gegebenenfalls kann der in Schritt (4) oder (4) (a) erhaltene Extrakt vor dem Durchführen des Schritt (5) filtriert werden. Das Filtrieren kann durch:
(a) Mikrofiltration und/oder
(b) Ultrafiltration durchgeführt werden.

Bei der Mikrofiltration werden Mikroteilchen mit einer Korngröße von etwa 0,1 bis etwa 10 µm mit Hilfe eines Membranfilters abgetrennt. Bei der Ultrafiltration werden makromolekulare Stoffe mittels eines Membranfilters abgetrennt. Der Cut-Off des Membranfilters ist vorzugsweise von 5000 bis 100.000 Dalton, insbesondere 10.000 Dalton. Außerdem kann mit dem Retenat der Mikrofiltration oder der Ultrafiltration eine Diafiltration durchgeführt werden.

Vorzugsweise wird der Extrakt vor dem Durchführen des Schritts (5) zuerst einer Mikrofiltration und sodann einer Ultrafiltration unterworfen. Insbesondere kann zusätzlich das Retenat der Mikrofiltration einer Diafiltration unterworfen werden, wobei ein Filtrat erhalten wird, das wiederum der Ultrafiltration unterworfen wird. Außerdem kann auch das bei der Ultrafiltration erhaltene Retenat wiederum einer Diafiltration unterworfen werden, wobei das dadurch erhaltene Filtrat weiter verarbeitet wird. Die Diafiltration wird vorzugsweise bei einer Temperatur von etwa 45°C durchgeführt.

Durch das erfindungsgemäße Verfahren erhält man einen Trockenextrakt, der frei von Rückständen organischer Lösungsmittel ist und verbesserte Färbeeigenschaften aufweist.

Des weiteren ist Gegenstand der vorliegenden Erfindung eine Emulsion, die (A) eine Fettphase, und (B) eine wäßrige Phase, die einen erfindungsgemäßen Trokkenextrakt enthält, umfaßt.

Die Fettphase (A) kann aus nativen Ölen, Esterölen und Wachsen bestehen. Bevorzugt verwendete Öle sind süßes Mandelöl, Avocadoöl, Kokosöl und Olivenöl. Bevorzugt verwendete Esteröle sind Linofeate, Oleate, Lactate und Glycerinester. Bevorzugt verwendete Wachse sind Bienenwachs, Carnaubawachs, Candelillawachs und Kakaobutter. Außerdem können Hilfsmittel, wie Laurylglykoside und Polyglyceryl-2-polyhydroxystearate in der Fettphase (A) enthalten sein.

Die wäßrige Phase (B) besteht vorzugsweise aus Wasser, wobei der Wassergehalt, bezogen auf die Gesamtmenge der Emulsion, vorzugsweise im Bereich von 40 bis 60% liegt. Außerdem kann die wäßrige Phase (B) Glycerin enthalten, wobei der Gehalt an Glycerin, bezogen auf die Gesamtmenge an Emulsion, im Bereich von etwa 1 bis 5% liegt. Die wäßrige Phase (B) kann außerdem als zusätzlichen Bestandteil einen Lösungsvermittler enthalten. Bevorzugte Lösungsvermittler sind Polyglyceryl-4-caprat (PGC), Polyglyceryl-3-laurate, Glycerylcaprylate und Polyglyceryl-6-dicaprate.

Die Menge an eingesetztem Trockenextrakt, bezogen auf die Gesamtmenge an Emulsion, beträgt vorzugsweise 0,5 bis 5, insbesondere 2 bis 3 Gew.-%.

Die erfindungsgemäße Emulsion kann zusätzlich ein Verdickungsmittel (C) enthalten. Bevorzugte Verdickungsmittel sind Magnesium-Aluminium-Silicat, Xanthan Gum, Alginat, Cellulose Gum und Siliciumdioxid.

Des weiteren kann die erfindungsgemäße Emulsion eine Säure oder Base enthalten. Die Säure oder Base wird in einer solchen Menge verwendet, daß die Emulsion einen pH-Wert von etwa 4 bis etwa 6, vorzugsweise etwa 5 hat. Bevorzugte Säuren sind Citronensäure, Milchsäure und Phytinsäure. Bevorzugte Basen sind NaOH und KOH.

Die erfindungsgemäße Emulsion kann zusätzlich übliche Hilfsstoffe, z.B. Duftstoffe, wie Rosenöl oder andere ätherische Öle, Antioxidantien, wie Tocopherol, und Pflegestoffe, wie Panthenol oder Weizen- oder Seidenhydrolysate enthalten.

Außerdem kann die erfindungsgemäße Emulsion weitere Farbstoffe, wie Juglon, enthalten.

Der Gehalt an Fettphase (A), bezogen auf die Gesamtmenge an Emulsion, beträgt vorzugsweise 10 bis 60, insbesondere 20 bis 40 Gew.-%. Die Menge an wäßriger Phase (B), bezogen auf die Gesamtmenge an Emulsion, beträgt vorzugsweise 50 bis 90, insbesondere 60 bis 80 Gew.-%. Die Menge an Lösungsvermittler, bezogen auf die Gesamtmenge an Emulsion, beträgt vorzugsweise 2 bis 20, insbesondere 6 bis 12 Gew.-%. Die Menge an Verdickungsmittel (C), bezogen auf die Gesamtmenge an Emulsion, beträgt vorzugsweise 0,5 bis 15, insbesondere 4 bis 12 Gew.-%.

Die erfindungsgemäße Emulsion kann hergestellt werden durch Erwärmen der Fettphase (A), Erwärmen der wäßrigen Phase (B) und Zugeben des Trockenextrakts zur wäßrigen Phase (B) unter starkem Rühren. Anschließend wird die Phase (B) in die Phase (A) einemulgiert und unter Rühren erkalten lassen. Dadurch wird eine O/W-Emulsion (Öl in Wasser-Emulsion) erhalten. Gegebenenfalls kann anschließend der pH-Wert durch Zugabe einer Säure oder einer Base eingestellt werden. Außerdem kann der wäßrigen Phase vor dem Zugeben des Trockenextrakts ein Lösungsvermittler zugegeben werden. Die Temperatur, auf die die Phasen (A) und (B) vorzugsweise erwärmt werden, liegt zwischen etwa 60 und etwa 90°C, vorzugsweise bei etwa 75°C.

Der erfindungsgemäße Trockenextrakt kann unter anderem in der Pharmazie, Medizin oder Kosmetik Verwendung finden. Beispielsweise ist es bekannt, Lawson bei Sichelzellenanämie, Amöbenruhr, Magengeschwüren, oder als Febrifugum, oder äußerlich bei Hautkrankheiten und Verbrennungen und als Adstrigens oder Antiseptikum erfolgreich einzusetzen. Außerdem ist die bakteriostatische Wirksamkeit von Lawson bekannt.

Weiterhin kann der erfindungsgemäße Extrakt in Pflanzenbreis zur Färbung, insbesondere von Haaren, aber auch von Textilien oder zur Bemalung von Händen und Füßen verwendet werden.

Auch die erfindungsgemäßen Emulsionen können in den genannten Gebieten Einsatz finden. Vorzugsweise wird die erfindungsgemäße Emulsion beim Färben von Haaren verwendet. Sie hat dabei den Vorteil, daß sie leichter auftragbar ist, als ein Pflanzenbrei aus Hennablättern, und allgemein besser handhabbar.

Wird die Emulsion beim Färben von Haaren verwendet, ist die Konzentration des Trockenextrakts, bezogen auf das Gewicht der Emulsion, vorzugsweise 0,5 bis 5,0%, insbesondere 2,5%. Die Einwirkungsdauer der Emulsion auf die Haare liegt vorzugsweise im Bereich von etwa 15 Minuten bis etwa 3 Stunden und die Temperatur während des Färbevorgangs kann vorzugsweise Raumtemperatur, bevorzugter etwa 40 bis etwa 50 °C sein.

Nachstehend wird die Erfindung anhand von Beispielen erläutert.

### Beispiel 1

1 t geschnittene Hennablätter mit einem Schüttgewicht von etwa 300kg/m³ werden mit 3 t wäßriger HCl-Lösung mit einem pH-Wert von 2,5 gemischt und 30 Minuten bei Raumtemperatur gerührt. Sodann wird die erhaltene Maische über eine Bandpresse abgepreßt. Hierbei fallen etwa 2 t wäßrige Lösung an, die verworfen wird. Sodann wird der erhaltene Trester mit 10 t 0,15%iger wäßriger NaOH-Lösung 3 Stunden bei einer Temperatur von etwa 50°C unter Rühren extrahiert. Sodann wird das Gemisch abgepreßt und man erhält 10 t alkalischen Extrakt: Anschließend wird der Extrakt bei etwa 50°C auf einen Konzentrierungsgrad von 1:15 thermisch konzentriert. Der konzentrierte Extrakt wird auf eine Temperatur von 15°C gekühlt und der pH-Wert wird mit HCl-Lösung auf 2,5 eingestellt. Daraufhin fällt Lawsonschlamm aus und wird mittels eines Separators abgetrennt. Der erhaltene Lawsonschlamm wird getrocknet. Man erhält etwa 6% Trockenextrakt, bezogen auf die Ausgangsmenge an geschnittenen Hennablättern, mit einem Lawsongehalt von 15%.

### Beispiel 2

Die in Beispiel 1 verwendeten geschnittenen Hennablätter werden vor ihrer Verarbeitung gesiebt, wobei Staubbestandteile mit einem Durchmesser von < 0,25 mm abgetrennt werden. Sodann wird das Verfahren nach Beispiel 1 durchgeführt. Man erhält etwa 4,5% Trockenextrakt, bezogen auf die eingesetzte Menge an geschnittenen Hennablättern, mit einem Lawsongehalt von etwa 20%.

### Beispiel 3

Der nach der alkalischen Extraktion erhaltene, abgepreßte Trester nach Beispiel 1 wird mit 5 t Wasser bei etwa 50°C 2 Stunden lang extrahiert. Der Trester wird von dem erhaltenen Extrakt abgetrennt. Der so erhaltene Extrakt wird mit einem Extrakt, erhalten nach der alkalischen Extraktion nach Beispiel 1 vereinigt. Man erhält etwa 15 t Extrakt aus 1 t geschnittener Hennablätter mit einem pH-Wert von 7,5, einem Trockensubstanzgehalt von 1,2% und einem Lawsongehalt von 700 mg/l. Sodann wird der Extrakt nach Beispiel 1 weiterverarbeitet. Man erhält etwa 7% Trockenextrakt, bezogen auf die eingesetzte Menge an geschnittenen Hennablättern, mit einem Lawsongehalt von etwa 15%.

### Beispiel 4

Ein alkalischer Extrakt wird nach Beispiel 1 hergestellt. Zur Abtrennung grober Bestandteile, wird der Extrakt stehen gelassen, damit diese sedimentieren. Der Überstand wird dekantiert. Der so behandelte Überstand wird mit einem Cut-Off von 10.000 Dalton ultrafiltriert. Dem Retenat der Ultrafiltration wird Wasser zur Diafiltration zugegeben, um noch vorhandenes Lawson aus dem Retenat zu lösen. Die Diafiltration findet bei einer Temperatur von etwa 45°C statt. Das Filtrat der Ultrafiltration und der Diafiltration werden vereinigt und wie in Beispiel 1 weiterverarbeitet. Man erhält 2,5% Trockenextrakt, bezogen auf die eingesetzte Menge an Trockenextrakt, mit einem Lawsongehalt von 40%.

### Beispiel 5

Herstellung einer O/W-Emulsion, enthaltend einen Trockenextrakt nach Beispiel 4.

45,5 g Wasser und 12,0 g Polyglyceryl-4-caprat (PGC) werden gemischt und auf 75°C erwärmt. 2,5 g Trockenextrakt werden sukzessive zugegeben und das Gemisch wird anschließend 3 Minuten mit einem Ultra Turrax homogenisiert. Man erhält eine Phase (B).

25,0 g Olivenöl, 5,0 g Bienenwachs und 10 g IMWITOR® 375 (ein Gemisch aus Glycerylcitrat/Lactat/Linoleat/Oleat, erhältlich von Hüls AG) werden gemischt und auf 75°C erwärmt. Man erhält eine Phase (A). Die Phase (B) wird in die Phase (A) einemulgiert und das Gemisch unter Rühren erkalten gelassen. Anschließend wird soviel 10%ige Citronensäure zugegeben, daß der pH-Wert der Emulsion 5 beträgt.

### Beispiel 6

Die Emulsion wurde analog Beispiel 1 hergestellt, außer das für die Phase (A) 9,0 g Cetylalkohol, 18,0 g Olivenöl, 10,0 g IMWITOR® 375, 1,0 g Kakaobutter und 2,5 g Bienenwachs, und für die Herstellung der Phase (B) 2,5 g Trockenextrakt, 12,0 g PGC, 3,0 g Glycerin und 42,5 g Wasser verwendet werden.

### Beispiel 7

Die Herstellung der Emulsion erfolgt analog Beispiel 1, außer das für die Phase (A) 5,0 g Stearylalkohol, 15,0 g Olivenöl, 8,0 g IMWITOR® 375, 3,0 g Avocadoöl, 2,0 g Bienenwachs und 1,5 g Polyglyceryl-2-polyhydroxystearat, und für die Herstellung der Phase (B) 3,0 g Trockenextrakt, 14,5 g PGC, 3,0 g Glycerin und 45,0 g Wasser verwendet wurden.

### Beispiel 8

41,5 g Wasser werden auf 80°C erwärmt und 4g Veegum® (ein aus natürlichen Mineralvorkommen hergestelltes, komplexes kolloidales Magnesium-Aluminium-Silicat) wird unter Rühren zugefügt. Das Gemisch wird 1 Stunde heiß quellen gelassen. Man erhält eine Phase (C). Außerdem werden 10,0 g Wasser und 12,0 g PCG auf 75°C erwärmt und 2,5 g Trockenextrakt nach Beispiel 4 werden unter starkem Rühren zugegeben und das Gemisch wird 3 Minuten mit einem Ultra Turrax homogenisiert. Sodann werden 2,0 g Glycerin zugegeben. Außerdem werden 9,0 g Olivenöl, 2,0 g Bienenwachs, 4,0 g Avocadoöl, 6,0 g IMWITOR® 375 und 1,0 g Polyglyceryl-2-polyhydroxystearat gemischt und auf 75°C erwärmt. Man erhält eine Phase (A).

Die Phase (B) wird in die Phase (C) eingerührt, und anschließend wird die Phase (A) einemulgiert und das Gemisch unter langsamen Rühren erkalten gelassen. Anschließend wird der pH-Wert der Emulsion mit 10%iger Citronensäure auf 5,0 eingestellt.

### Beispiel 9

Die Herstellung der Emulsion erfolgt analog Beispiel 8, außer das für die Phase (A) 9,0 g Olivenöl, 4,0 g Bienenwachs, 4,0 g Avocadoöl, 6,5 g IMWITOR® 375 und 1,0 g Polyglyceryl-2-polyhydroxystearat, und für die Herstellung der Phase (B) 2,5 g Trockenextrakt, 12,0 g PGC, 2,0 g Glycerin und 10,0 g Wasser, und für die Herstellung der Phase (C) 6,0 g Veegum® und 36,0 g Wasser verwendet wurden. Außerdem wurde die Phase (C) 2 Stunden quellen gelassen.

### Beispiel 10

Die Herstellung der Emulsion erfolgt analog Beispiel 8, außer das für die Phase (A) 9,0 g Olivenöl, 4,0 g Bienenwachs, 4,0 g Avocadoöl, 6,5 g IMWITOR® 375 und 1,0 g Polyglyceryl-2-polyhydroxystearat, und für die Herstellung der Phase (B) 2,5 g Trockenextrakt, 12,0 g Polyglyceryl-4-caprat, 2,0 g Glycerin und 10,0 g Wasser, und für die Herstellung der Phase (C) 6,0 g Veegum®, 2,0 g Xanthan Gum und 41,0 g Wasser verwendet wurden.

### Beispiel 11

48,0 g Wasser und 12,0 g PGC werden gemischt und auf 75°C erwärmt. 2,5 g Trokkenextrakt werden unter starkem Rühren zugegeben und das Gemisch wird sodann 3 Minuten mit einem Ultra Turrax homogenisiert. Sodann werden 2,0 g Glycerin zugefügt. Man erhält eine Phase (B). Außerdem werden 6,0 g süßes Mandelöl, 3,0 Bienenwachs, 4,0 g Avocadoöl, 5,0 g Kokosöl, 1,0 g Laurylglykosid, 5,5 g IMWITOR® 375 und 1,0 g Polyglyceryl-2-polyhydroxystearat gemischt und auf 75°C erwärmt. Man erhält eine Phase (A). Die Phase (A) wird in die Phase (B) einemulgiert und anschließend 1 Minute homogenisiert. Als Phase (C) werden 10,0 g Aerosil® 200 (ein kolloidales Siliciumdioxid) zugefügt, bis das Pulver vollständig eingearbeitet ist. Anschließend wird der pH-Wert der Emulsion mit NaOH auf 5,0 eingestellt.

### Beispiel 12

Die Herstellung der Emulsion erfolgte analog Beispiel 11, außer daß für die Herstellung der Phase (A) 4,0 g süßes Mandelöl, 3,0 Bienenwachs, 3,0 g Avocadoöl, 5,0 g Kokosöl, 3,0 g Laurylglykosid, 4,5 g IMWITOR® 375 und 1,0 g Polyglyceryl-2-polyhydroxystearat, für die Herstellung der Phase (B) 2,0 Trockenextrakt, 12,0 g PGC, 2,0 g Glycerin und 51,0 Wasser verwendet wurden. Außerdem wurde vor dem Einstellen des pH-Wertes ein Gemisch aus 1,0 g CK 15 (eine ätherische Ölmischung, erhältlich von der Firma Logona), 0,3 g Tocopherol, 1,0 g α-Rosenöl und 0,2 g Panthenol zugegeben.

### Beispiel 13

Die Herstellung der Emulsion erfolgte analog Beispiel 12, außer daß bei der Herstellung der Phase (A) 2,0 g Laurylglykosid, bei der Herstellung der Phase (B) 52,5 g Wasser verwendet wurden und 0,5 g α-Rosenöl zugegeben wurden.

Die Emulsion, die nach Beispiel 13 hergestellt wurde, ist eine dunkel rot-braun gefärbte Creme. Sie besitzt eine streichförmige Konsistenz und ist leicht auf das Haar aufzutragen und wieder auszuwaschen. Nach der Anfärbung fühlen sich die Haare geschmeidiger an, als die mit Pflanzenpulver angefärbten Haare und sind leichter kämmbar. Die Creme hat außerdem einen angenehmen Geruch.

Die physikalische Stabilität der Emulsion nach Beispiel 13 wurde wie nachstehend getestet:

### Experimentelle Bedingungen:

Geprüft wurde über einen Zeitraum von 2 Wochen bei folgenden Temperaturen: + 20 °C, + 40°C, + 20°C, - 5°C. Der Temperaturwechsel erfolgte jeweils nach 24 Stunden.

### Ergebnis:

Die Emulsion zeigte nach 2 Wochen keine Phasentrennung. Die Anfärbung von BBG-Strähnen (gebleichte Strähnen vom Büffelbauch) entsprach dem Färbeergebnis vor der Prüfung.

Außerdem wurde die mikrobiologische Stabilität der Emulsion getestet.

Die mikrobiologische Stabilität ist eine Meßgröße dafür, ob die Emulsion den Anforderungen an die Prüfung auf ausreichende Konservierung gemäß Ph. Eur. 1997 entspricht.

Ergebnisse der mikrobiologischen Stabilität:

| Keimzahlbestimmung | Ergebnis | entspricht | |
|---|---|---|---|
| | | ja | nein |
| Kolonienzahl/g bzw. ml Bakterien | < 10 | X | |
| Kolonienzahl/g bzw. ml Hefen/Schimmelpilze | < 10 | X | |
| Differenzierung erfolgt: ja [X] nein [ ] | | | |

| | Grenzwert | Ergebnis | entspricht | |
|---|---|---|---|---|
| | | | ja | nein |
| Enterobakterien | | n.n. | X | |
| Escherichia coli | n.n.* | n.n. | X | |
| Staphylococcus aureus | n.n.* | n.n. | X | |
| Pseudomonas aeruginosa | n.n.* | n.n. | X | |
| n.n. = nicht nachweisbar | | | | |

Außerdem wurde die Färbeeigenschaft der Emulsion nach Beispiel 13 bestimmt.

Da sich die resultierende Färbung immer additiv aus der Eigenhaarfarbe und dem eingesetzten Färbemittel zusammensetzt, wurde zur Beurteilung des Färbeergebnisses Humanhaar verwendet. Es wurde unbehandeltes europäisches Naturhaar der Haarfarbe mittelbraun, hellbraun, dunkelblond, mittelblond und hellblond eingesetzt.

### Durchführung:

In die zuvor mit handelsüblichen Shampoo gewaschenen Haarsträhnen wurde jeweils die Emulsion nach Beispiel 13 eingekämmt. Die Strähnen wurden mit Aluminiumfolie umwickeit und bei 40°C 1 Stunde im Wärmeofen entwickelt. Anschließend wurden die Strähnen wiederum gewaschen und das Färbeergebnis optisch ausgewertet.

Als Referenz dienten einerseits die unbehandelten Euro-Naturhaare und zum anderen mit Henna-Pflanzenpulver angefärbte der jeweiligen Haarfarbe. Die Anfärbung mit Henna-Pflanzenpulver erfolgte als Kataplasmafärbung, wobei 100 g Henna-Pflanzenpulver in 300 ml kochendem Wasser zu einem homogenen Brei verarbeitet wurden und nach Abkühlung auf etwa 60°C auf die Strähnen aufgetragen wurde.

Die weitere Behandlung erfolgte analog der Anfärbung mit der Emulsion nach Beispiel 13.

Die Ergebnisse sind in der Tabelle 1 zusammengefaßt:

**Tabelle 1:**

| Naturhaarfarbe | Färbeergebnis | |
|---|---|---|
| | a) Hennapulver | b) Färbecreme |
| Mittelbraun | leichter Rotreflex | leichter Rotreflex Rotreflex schwächer als a) Grundton dunkler als a) |
| Hellbraun | dunkle Kirsche | dunkle Kirsche Färbung geringfügig schwächer als a) |
| Dunkelblond | Kastanie | Kastanie kein Unterschied zu a) |
| Mittelblond | dunkles Kupfer | dunkles Kupfer kein Unterschied zu a) |
| Hellblond | Kupfer | Kupfer kein Unterschied zu a) |

### Beispiel 14

Zusammensetzung einer Haarfärbecreme mit einem Trockenextrakt nach Beispiel 4:

| | **Komponente** | **Menge [kg]** |
|---|---|---|
| **Phase A:** | Mandelöl | 5,5 |
| | gelbes Bienenwachs | 3,0 |
| | Avocadoöl | 3,0 |
| | Kokosöl | 11,0 |
| | IMWITOR® 375 (Glycerylcitrate/Lactat/Linoleat/Oleat) | 4,5 |
| | DEHYMULS® PGPH (Polyglyceryl-2-polyhydroxystearat) | 1,0 |
| **Phase B:** | Trockenextrakt (Beispiel 4) | 1,6 |
| | DERMOFEEL® 81 F (Polyglyceryl-6-dicaprat [100%]) | 6,0 |
| | Glycerin 99,5% DAB 10 | 4,0 |
| | Wasser, gereinigt | 54,4 |
| **Phase C:** | AEROSIL® 200 (Siliciumdioxid) | 4,0 |
| **Hilfsstoffe:** | CK 15 | 1,0 |
| | Tocopherol | 0,3 |
| | PARF. ROSE ALPHA 222.251 (Duftstoffe [83%], Alkohol [17%]) | 0,5 |
| | D-Panthenol DAB | 0,2 |

## Patentansprüche

1. Trockenextrakt aus der Hennapflanze oder Teilen davon, enthaltend 10 bis 60 Gew.-% Lawson (2-Hydroxy-1,4-naphthochinon), erhältlich durch:
(1) Mischen des Pflanzenmaterials der Hennapflanze oder Teilen davon mit einer sauren wässrigen Lösung, wodurch eine Maische erhalten wird,
(2) Trennen der in Schritt (1) erhaltenen Maische in einen Trester und eine wässrige Lösung,
(3) Extrahieren des in Schritt (2) erhaltenen Tresters mit einer alkalischen wässrigen Lösung,
(4) Abtrennen des in Schritt (3) erhaltenen Extrakts von dem Trester,
(5) Konzentrieren des in Schritt (4) erhaltenen Extrakts,
(6) Abkühlen und Ansäuem des in Schritt (5) erhaltenen Extrakts, wodurch ein Lawsonschlamm erhalten wird, und
(7) Abtrennen und Trocknen des in Schritt (6) erhaltenen Lawsonschlamms.

2. Trockenextrakt nach Anspruch 1, enthaltend 20 bis 45 Gew.-% Lawson.

3. Trockenextrakt nach Anspruch 2, enthaltend 35 bis 42 Gew.-% Lawson.

4. Trockenextrakt nach einem der Ansprüche 1 bis 3, **dadurch gekennzeichnet, dass** der Teil der Hennapflanze deren Blätter sind.

5. Trockenextrakt nach Anspruch 4, **dadurch gekennzeichnet, dass** die Blätter zerkleinert sind.

6. Verfahren zur Herstellung des Trockenextrakts nach einem der Ansprüche 1 bis 5, dass die Schritte umfasst:
(1) Mischen des Pflanzenmaterials der Hennapflanze oder Teilen davon mit einer sauren wässrigen Lösung, wodurch eine Maische erhalten wird,
(2) Trennen der in Schritt (1) erhaltenen Maische in einen Trester und eine wässrige Lösung,
(3) Extrahieren des in Schritt (2) erhaltenen Tresters mit einer alkalischen wässrigen Lösung,
(4) Abtrennen des in Schritt (3) erhaltenen Extrakts von dem Trester,
(5) Konzentrieren des in Schritt (4) erhaltenen Extrakts,
(6) Abkühlen und Ansäuern des in Schritt (5) erhaltenen Extrakts, wodurch ein Lawsonschlamm erhalten wird, und
(7) Abtrennen und Trocknen des in Schritt (6) erhaltenen Lawsonschlamms.

7. Verfahren nach Anspruch 6, **dadurch gekennzeichnet, dass** der in Schritt (4) abgetrennte Trester in einem Schritt (4) (a) mit Wasser nachextrahiert wird.

8. Verfahren nach einem der Ansprüche 6 oder 7, **dadurch gekennzeichnet, dass** der nach Schritt (4) oder Schritt (4) (a) erhaltene Extrakt vor Schritt (5) mindestens einem der Schritte
(a) Mikrofiltration und/oder
(b) Ultrafiltration
unterworfen wird.

9. Verfahren nach einem der Ansprüche 6 bis 8, **dadurch gekennzeichnet, dass**, die saure wässrige Lösung in Schritt (1) einen pH-Wert von 1 bis 6, vorzugsweise etwa 2,5 hat.

10. Verfahren nach einem der Ansprüche 6 bis 9, **dadurch gekennzeichnet, dass**, die alkalische wässrige Lösung in Schritt (3) eine 0,15%ige NaOH-Lösung ist.

11. Verfahren nach einem der Ansprüche 6 bis 10, **dadurch gekennzeichnet, dass** in Schritt (1) das Verhältnis von Pflanzenmaterial der Hennapflanze zu wässriger Lösung 1:3 ist und dieser Schritt bei einer Temperatur von etwa 20°C für einen Zeitraum von etwa 0,5 Stunden durchgeführt wird.

12. Verfahren nach einem der Ansprüche 6 bis 11, **dadurch gekennzeichnet, dass** in Schritt (3) das Verhältnis von Trester zu wässriger Lösung 1:10, bezogen auf die Menge an eingesetztem Pflanzenmaterial der Hennapflanze, ist und dieser Schritt bei einer Temperatur von etwa 50°C für einen Zeitraum von etwa 3 Stunden durchgeführt wird.

13. Verfahren nach einem der Ansprüche 7 bis 12, **dadurch gekennzeichnet, dass** in Schritt (4) das Verhältnis von Trester zu Wasser 1:5, bezogen auf die Menge an eingesetztem Pflanzenmaterial der Hennapflanze, ist und dieser Schritt bei einer Temperatur von etwa 50°C für einen Zeitraum von etwa 2 Stunden durchgeführt wird.

14. Verfahren nach einem der Ansprüche 6 bis 13, **dadurch gekennzeichnet, dass** das Konzentrieren in Schritt (5) durch Umkehrosmose oder thermisches Konzentrieren durchgeführt wird.

15. Emulsion, umfassend
(A) eine Fettphase und
(B) eine wässrige Phase, die einen Trockenextrakt aus der Hennapflanze oder Teilen davon, enthaltend 10 bis 60 Gew.-% Lawson (2-Hydroxy-1,4-naphthochinon), enthält.

16. Emulsion nach Anspruch 15, **dadurch gekennzeichnet, dass** zusätzlich ein Lösungsvermittler enthalten ist.

17. Emulsion nach Anspruch 16, **dadurch gekennzeichnet, dass** der Lösungsvermittler Polyglyceryl-6-dicaprat ist.

18. Emulsion nach einem der Ansprüche 15 bis 17, **dadurch gekennzeichnet, dass** zusätzlich ein Verdickungsmittel (C) enthalten ist.

19. Emulsion nach einem der Ansprüche 15 bis 18, **dadurch gekennzeichnet, dass** zusätzlich weitere natürliche Farbstoffe enthalten sind.

20. Emulsion nach einem der Ansprüche 15 bis 19, **dadurch gekennzeichnet, dass** der Gehalt an Trockenextrakt von 0,5 bis 5 Gew.-% bezogen auf das Gewicht der Emulsion, ist.

21. Verwendung des Trockenextrakts nach einem der Ansprüche 1 bis 5 als Kosmetikum, Haarfärbemittel oder in einer Flüssigkeit zur Hautbemalung.

22. Trockenextrakt nach einem der Ansprüche 1 bis 5 zur Verwendung als Arzneimittel.

23. Verwendung der Emulsion nach einem der Ansprüche 15 bis 20 als Kosmetikum, Haarfärbemittel oder zur Hautbemalung.

24. Emulsion nach einem der Ansprüche 15 bis 20 zur Verwendung als Arzneimittel.

## Claims

1. Dry extract of the henna plant or parts thereof containing 10 to 60 %wt lawsone (2-hydroxy-1,4-naphthoquinone), obtainable by:
(1) mixing the plant material of the henna plant or parts thereof with an acid aqueous solution to obtain a mash,
(2) separation of the mash obtained in step (1) into a cake and an aqueous solution,
(3) extraction of the cake obtained in step (2) with an alkaline aqueous solution,
(4) separation of the extract obtained in step (3) from the cake,
(5) concentration of the extract obtained in step (4),
(6) cooling and acidulation of the extract obtained in step (5) to yield a lawsone sludge, and
(7) separation and drying of the lawsone sludge obtained in step (6).

2. Dry extract according to Claim 1 containing 20 to 45 % lawsone.

3. Dry extract according to Claim 2 containing 35 to 42 % lawsone.

4. Dry extract according to any one of Claims 1 to 3, **characterized in that** the part of the henna plant is its leaves.

5. Dry extract according to Claim 4, **characterized in that** the leaves are shredded.

6. Process for the preparation of the dry extract according to any one of Claims 1 to 5, comprising the following steps:
(1) mixing the plant material of the henna plant or parts thereof with an acid aqueous solution to obtain a mash,
(2) separation of the mash obtained in step (1) into a cake and an aqueous solution,
(3) extraction of the cake obtained in step (2) with an alkaline aqueous solution,
(4) separation of the extract obtained in step (3) from the cake,
(5) concentration of the extract obtained in step (4),
(6) cooling and acidulation of the extract obtained in step (5) to yield a lawsone sludge, and
(7) separation and drying of the lawsone sludge obtained in step (6).

7. Process according to Claim 6, **characterized in that** the cake separated in step (4) is reextracted with water in a step (4) (a).

8. Process according to Claim 6 or Claim 7, **characterized in that** the extract obtained by step (4) or step (4) (a) is subjected before step (5) to at least one of the steps of
(a) microfiltration and/or
(b) ultrafiltration.

9. Process according to any one of Claims 6 to 8, **characterized in that** the acid aqueous solution in step (1) has a pH of 1 to 6 and preferably of about 2.5.

10. Process according to any one of Claims 6 to 9, **characterized in that** the alkaline aqueous solution in step (3) is a 0.15% NaOH solution.

11. Process according to any one of Claims 6 to 10, **characterized in that** the proportion of plant material of the henna plant to aqueous solution in step (1) is 1:3, and this step is carried out at a temperature of approximately 20°C for a period of approximately 0.5 hour.

12. Process according to any one of Claims 6 to 11, **characterized in that** the proportion of cake to aqueous solution in step (3), in terms of the quantity of plant material of the henna plant used, is 1:10, and this step is carried out at a temperature of approximately 50°C for a period of approximately 3 hours.

13. Process according to any one of Claims 7 to 12, **characterized in that** the proportion of cake to water in step (4), in terms of the quantity of plant material of the henna plant used, is 1:5, and this step is carried out at a temperature of approximately 50°C for a period of approximately 2 hours.

14. Process according to any one of Claims 6 to 13, **characterized in that** the concentration in step (5) is performed by reverse osmosis or by thermal concentration.

15. Emulsion comprising
(A) a fat phase and
(B) an aqueous phase which contains a dry extract of the henna plant or parts thereof containing 10 to 60 %wt lawsone (2-hydroxy-1,4-naphthoquinone).

16. Emulsion according to Claim 15, **characterized in that** a solubilizing agent is additionally contained.

17. Emulsion according to Claim 16, **characterized in that** the solubilizing agent is polyglyceryl-6-dicaprate.

18. Emulsion according to any one of Claims 15 to 17, **characterized in that** a thickening agent (C) is additionally contained.

19. Emulsion according to any one of Claims 15 to 18, **characterized in that** further natural dyes are additionally contained.

20. Emulsion according to any one of Claims 15 to 19, **characterized in that** the dry extract content is from 0.5 to 5 %wt of the emulsion.

21. Use of the dry extract according to any one of Claims 1 to 5 as a cosmetic or hair dye, or in a liquid for body painting.

22. Dry extract according to any one of Claims 1 to 5 for use as a medicament.

23. Use of the emulsion according to any one of Claims 15 to 20 as a cosmetic or hair dye, or in a liquid for body painting.

24. Emulsion according to any one of Claims 15 to 20 for use as a medicament.

## Revendications

1. Extrait sec de plante de henné ou de parties de celle-ci, contenant de 10 à 60 % en poids de lawsone (2-hydroxy-1,4-naphtoquinone), pouvant être obtenu par les étapes consistant à :
(1) mélanger la matière végétale de la plante de henné ou des parties de celle-ci avec une solution aqueuse acide, ce qui permet d'obtenir un moût,
(2) séparer le moût obtenu à l'étape (1) en un marc et en une solution aqueuse,
(3) extraire le marc obtenu à l'étape (2) avec une solution aqueuse alcaline,
(4) séparer l'extrait obtenu à l'étape (3) du marc,
(5) concentrer l'extrait obtenu à l'étape (4),
(6) refroidir et acidifier l'extrait obtenu à l'étape (5), ce qui permet d'obtenir une boue de Lawsone, et
(7) séparer et sécher la boue de Lawsone obtenue à l'étape (6).

2. Extrait sec selon la revendication 1, contenant de 20 à 45 % en poids de Lawsone.

3. Extrait sec selon la revendication 2, contenant de 35 à 42 % en poids de Lawsone.

4. Extrait sec selon l'une quelconque des revendications 1 à 3, **caractérisé en ce que** la partie de la plante de henné est constituée de ses feuilles.

5. Extrait sec selon la revendication 4, **caractérisé en ce que** les feuilles sont broyées.

6. Procédé de production de l'extrait sec selon l'une quelconque des revendications 1 à 5, **caractérisée par** les étapes consistant à :
(1) mélanger la matière végétale de la plante de henné ou des parties de celle-ci avec une solution aqueuse acide, ce qui permet d'obtenir un moût,
(2) séparer le moût obtenu à l'étape (1) en un marc et en une solution aqueuse,
(3) extraire le marc obtenu à l'étape (2) avec une solution aqueuse alcaline,
(4) séparer l'extrait obtenu à l'étape (3) du marc,
(5) concentrer l'extrait obtenu à l'étape (4),
(6) refroidir et acidifier l'extrait obtenu à l'étape (5), ce qui permet d'obtenir une boue de Lawsone, et
(7) séparer et sécher la boue de Lawsone obtenue à l'étape (6).

7. Procédé selon la revendication 6, **caractérisé en ce que** le marc séparé à l'étape (4) est encore extrait dans une étape (4)(a) avec l'eau.

8. Procédé selon l'une quelconque des revendications 6 ou 7, **caractérisé en ce que** l'extrait obtenu après l'étape (4) ou l'étape (4)(a) est soumis à l'une des étapes suivantes avant l'étape (5) :
(a) microfiltration et / ou
(b) ultrafiltration.

9. Procédé selon l'une quelconque des revendications 6 à 8, **caractérisé en ce que**, à l'étape (1), la solution aqueuse acide a un pH compris entre 1 et 6, de préférence égal à environ 2,5.

10. Procédé selon l'une quelconque des revendications 6 à 9, **caractérisé en ce que**, à l'étape (3), la solution aqueuse alcaline est une solution de NaOH à 0,15 %.

11. Procédé selon l'une quelconque des revendications 6 à 10, **caractérisé en ce que**, à l'étape (1), le rapport entre la matière végétale de la plante de henné et la solution aqueuse est de 1/3, et que cette étape est réalisée à une température d'environ 20° C pendant environ 0,5 heure.

12. Procédé selon l'une quelconque des revendications 6 à 11, **caractérisé en ce que**, à l'étape (3), le rapport entre le marc et la solution aqueuse est de 1/10, par rapport à la quantité de la matière végétale utilisée de la plante, et cette étape est réalisée à une température d'environ 50° C pendant 3 heures.

13. Procédé selon l'une quelconque des revendications 7 à 12, **caractérisé en ce que**, à l'étape (4), le rapport entre le marc et l'eau est de 1/5, par rapport à la quantité de matière végétale utilisée de la plante de henné, et que cette étape est réalisée à une température d'environ 50° C pendant environ deux heures.

14. Procédé selon l'une quelconque des revendications 6 à 13, **caractérisé en ce que** la concentration à l'étape (5) est réalisée par osmose inverse ou concentration thermique.

15. Emulsion, comprenant :
(A) une phase grasse et
(B) une phase aqueuse, qui contient un extrait sec de la plante de henné ou des parties de celle-ci, contenant de 10 à 60 % en poids de lawsone (2-hydroxy-1,4-naphtoquinone).

16. Emulsion selon la revendication 15, **caractérisée en ce qu'**il est en outre prévu un agent de solubilisation.

17. Emulsion selon la revendication 16, **caractérisée en ce que** l'agent de solubilisation est le polyglycéryl-6-dicaprate.

18. Emulsion selon l'une quelconque des revendications 15 à 17, **caractérisée en ce qu'**elle contient en outre un agent épaississant (C).

19. Emulsion selon l'une quelconque des revendications 15 à 18, **caractérisée en ce qu'**elle contient en outre d'autres colorants naturels.

20. Emulsion selon l'une quelconque des revendications 15 à 19, **caractérisée en ce que** la teneur en extrait sec est comprise entre 0,5 et 5 % en poids par rapport au poids de l'émulsion.

21. Utilisation de l'extrait sec selon l'une quelconque des revendications 1 à 5 en tant que cosmétique, teinture pour les cheveux ou dans un liquide de coloration pour la peau.

22. Extrait sec selon l'une quelconque des revendications 1 à 5, destinée à être utilisée comme médicament.

23. Utilisation de l'émulsion selon l'une des revendications 15 à 20 en tant de produit cosmétique, pour teindre les cheveux ou colorer la peau.

24. Emulsion selon l'une quelconque des revendications 15 à 20, destinée à être utilisée comme médicament.
